# EUROPÄISCHE PATENTANMELDUNG

(11) **EP 4 545 051 A1**
(43) Veröffentlichungstag der Anmeldung: **30.04.2025**
(21) Anmeldenummer: 24208458.0
(22) Anmeldetag: 23.10.2024
(51) Int. Cl.: A61F 2/30, A61F 2/38

(54) **AUGMENT FÜR EIN KNOCHENIMPLANTAT, SATZ VON AUGMENTEN**

(30) Priorität: 27.10.2023 DE 102023129788
(71) Anmelder: Aesculap AG, 78532 Tuttlingen (DE)
(72) Erfinder: Hirt, Martin, 78333 Stockach (DE)
(74) Vertreter: DREISS Patentanwälte PartG mbB

(57) **Zusammenfassung**

Augment (102) und Satz von Augmenten (102) für ein Knochenimplantat insbesondere für eine Tibia oder ein Femur, wobei das Augment (102) einen Körper (104) aufweist, wobei der Körper (104) eine Schnittstelle (106) zur Befestigung des Augments (102) an einem Plateau (202) des Knochenimplantats, eine Schnittstelle (108) zur Befestigung des Augments (102) am Knochen und eine Schnittstelle (110 )zu einem Befestigungselement (206), zur Verankerung des Knochenimplantats in einem Markraum eines Knochens, insbesondere der Tibia oder des Femurs, aufweist, die sich zwischen der Schnittstelle (106) zur Befestigung des Augments (102) am Plateau (202) und der Schnittstelle (108) zur Befestigung des Augments (102) am Knochen erstreckt, wobei der Körper (104) an der Schnittstelle (110) zum Befestigungselement (206) einen Hinterschnitt aufweist.

## Beschreibung

Die Erfindung geht von einem Augment für ein Knochenimplantat und einem Satz von Augmenten aus.

Durch das Augment gemäß dem unabhängigen Anspruch 1 wird eine verbesserte Zementfixierung erreichbar als bei herkömmlichen Augmenten.

Das Augment für ein Knochenimplantat insbesondere für eine Tibia oder ein Femur weist einen Körper auf, wobei der Körper eine Schnittstelle zur Befestigung des Augments an einem Plateau des Knochenimplantats, eine Schnittstelle zur

Befestigung des Augments am Knochen und eine Schnittstelle zu einem Befestigungselement zur Verankerung des Knochenimplantats in einem Markraum eines Knochens, insbesondere der Tibia oder des Femurs, aufweist, die sich zwischen der Schnittstelle zur Befestigung des Augments am Plateau und der Schnittstelle zur Befestigung des Augments am Knochen erstreckt, wobei der Körper an der Schnittstelle zum Befestigungselement einen Hinterschnitt aufweist. Knochenzement im Hinterschnitt verbessert die Zementfixierung.

Es kann vorgesehen sein, dass der Hinterschnitt einen konischen oder konkaven Verlauf des Körpers von der Schnittstelle zur Befestigung des Augments am Knochen zur Schnittstelle zur Befestigung des Augments am Plateau umfasst. Dies erleichtert die Fertigung des Augments.

Es kann vorgesehen sein, dass sich der Körper zwischen der Schnittstelle zur Befestigung des Augments am Plateau und der Schnittstelle zur Befestigung des Augments am Knochen in einer ersten Höhe erstreckt, wobei sich der Hinterschnitt zwischen der Schnittstelle zur Befestigung des Augments am Plateau und der Schnittstelle zur Befestigung des Augments am Knochen in einer zweiten Höhe erstreckt, die kleiner als die erste Höhe ist, insbesondere von der zweiten Höhe bis zur Schnittstelle zur Befestigung des Augments am Plateau.

Die Schnittstelle zur Befestigung des Augments am Knochen erstreckt sich in einer Ausführungsform im Wesentlichen in einer Ebene, wobei der Hinterschnitt eine Wand des Körpers umfasst, die zumindest abschnittsweise in einem Winkel zwischen 89° und 45°, insbesondere zwischen 89° und 79°, zur Ebene angeordnet ist.

Es kann vorgesehen sein, dass der Körper sich außerhalb der Schnittstelle zum Befestigungselement von der Schnittstelle zur Befestigung des Augments am Plateau zur Schnittstelle zur Befestigung des Augments am Knochen verjüngt oder dass die Schnittstelle zur Befestigung des Augments am Knochen sich im Wesentlichen in der Ebene erstreckt, wobei der Körper sich außerhalb der Schnittstelle zum Befestigungselement von der Schnittstelle zur Befestigung des Augments am Plateau zur Schnittstelle zur Befestigung des Augments am Knochen im Winkel von 90° oder im Wesentlichen von 90° zur Ebene erstreckt. Das bedeutet, der Hinterschnitt ist nicht an Oberflächen des Körpers angeordnet, die für die Zementfixierung nicht verwendet werden.

Es kann vorgesehen sein, dass der Körper sich von der Schnittstelle zur Befestigung des Augments am Plateau zur Schnittstelle zur Befestigung des Augments am Knochen verjüngt.

Der Körper kann eine zwischen der Schnittstelle zur Befestigung des Augments am Plateau und der Schnittstelle zur Befestigung des Augments am Knochen umlaufende Fläche umfasst, wobei die Fläche die Schnittstelle zum Befestigungselement umfasst. Der Hinterschnitt ist z.B. in die Fläche integriert.

Die Fläche weist beispielsweise an der Schnittstelle zum Befestigungselement zumindest abschnittsweise eine raue Oberfläche auf. Dadurch wird eine verbesserte Zementfixierung ermöglicht.

Die Fläche weist beispielsweise außerhalb der Schnittstelle zum Befestigungselement eine glatte Oberfläche auf. Das bedeutet, es wird keine raue Oberfläche an für die Zementfixierung unbenutzten stellen vorgesehen.

Die Schnittstelle zum Befestigungselement umfasst in einer Ausführungsform einen Teils eines Umfangs des Körpers, der sich zwischen der Schnittstelle zur Befestigung des Augments am Plateau und der Schnittstelle zur Befestigung des Augments am Knochen erstreckt, wobei sich der Hinterschnitt entlang des Teils des Umfangs insbesondere über den gesamten Teil des Umfangs erstreckt. Dadurch wird eine verbesserte Zementfixierung ermöglicht.

Es kann ein Satz von Augmenten vorgesehen sein, der wenigstens zwei der Augmente umfasst, wobei sich der Körper der wenigstens zwei Augmente zwischen der Schnittstelle zur Befestigung des jeweiligen Augments am Plateau und der Schnittstelle zur Befestigung des Augments am Knochen in unterschiedlichen Höhen erstreckt oder die Schnittstelle zum Befestigungselement für unterschiedlich große Befestigungselemente ausgebildet ist, oder sich die Hinterschnitte in ihrer Form oder im Winkel unterscheiden.

Es kann vorgesehen sein, dass der Satz von Augmenten ein erstes Augment und ein zweites Augment umfasst, wobei das erste Augment und das zweite Augment aneinander anordenbar sind, wobei die Schnittstelle zur Befestigung des ersten Augments am Plateau des Knochenimplantats und die Schnittstelle zur Befestigung des zweiten Augments am Knochen ausgebildet sind, das erste Augment und das zweite Augment zu verbinden.

Es kann vorgesehen sein, dass im Satz von Augmenten das zweite Augment ohne Hinterschnitt an seiner Schnittstelle zum Befestigungselement ausgebildet ist. Das bedeutet, das zweite Augment kann ein herkömmliches Augment sein.

Weitere vorteilhafte Ausführungsformen sind der folgenden Beschreibung und der Zeichnung entnehmbar. In der Zeichnung zeigt:
Fig. 1a eine schematische, perspektivische Ansicht eines Augments für ein Knochenimplantat für eine Tibia,
Fig. 1b eine Aufsicht auf das Augment für das Knochenimplantat für die Tibia,
Fig. 1c einen Schnitt durch das Augment für das Knochenimplantat für die Tibia,
Fig. 2 eine schematische, perspektivische Ansicht des Augments und wenigstens eines Teils des Knochenimplantats für die Tibia,
Fig. 3a eine Aufsicht auf ein Augment für ein Knochenimplantat für ein Femur,
Fig. 3b einen Schnitt durch das Augment für das Knochenimplantat für das Femur,
Fig. 4 eine schematische, perspektivische Ansicht des Augments für das Knochenimplantat für das Femur,
Fig. 5 eine schematische, perspektivische Ansicht des Augments und wenigstens eines Teils des Knochenimplantats für das Femur.

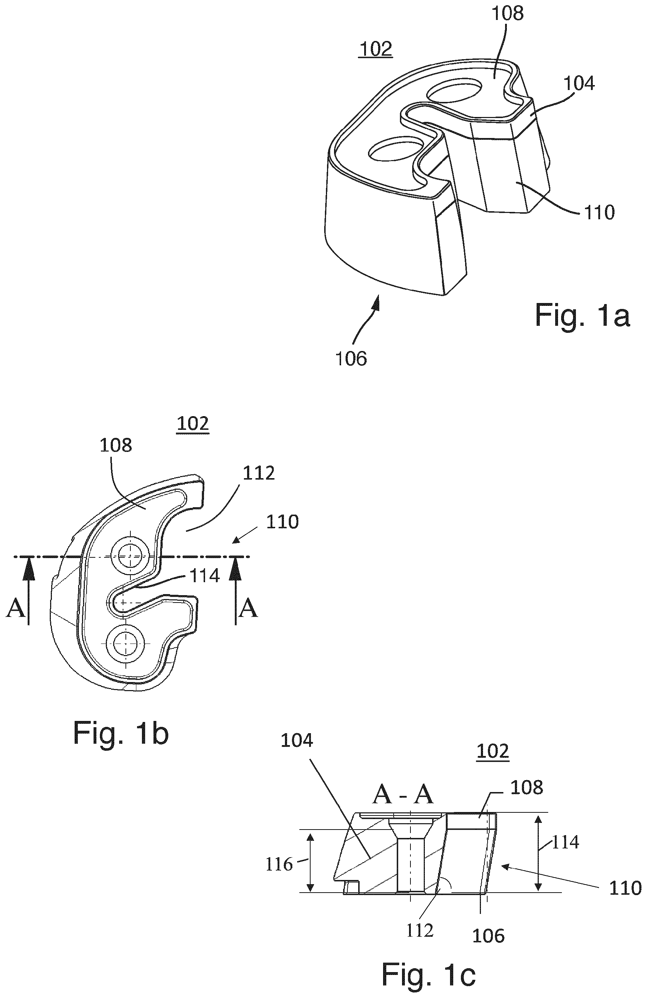

In Figur 1a ist eine schematische, perspektivische Ansicht eines Augments 102 für ein Knochenimplantat für eine Tibia dargestellt.

Das Augment 102 weist einen Körper 104 auf. Der Körper 104 weist eine erste Schnittstelle 106 auf. Die erste Schnittstelle 106 ist zur Befestigung des Augments 102 an einem Plateau des Knochenimplantats ausgebildet. Der Körper 104 weist eine zweite Schnittstelle 108 auf. Die zweite Schnittstelle 108 ist zur Befestigung des Augments 102 am Knochen ausgebildet. Der Körper 104 weist eine dritte Schnittstelle 110 zu einem Befestigungselement zur Verankerung des Knochenimplantats in einem Markraum eines Knochens auf.

Die dritte Schnittstelle 110 erstreckt sich zwischen der ersten Schnittstelle 106 und der zweiten Schnittstelle 108.

Der Körper 104 umfasst eine zwischen der ersten Schnittstelle 106 und der zweiten Schnittstelle 108 umlaufende Fläche. Die Fläche umfasst die dritte Schnittstelle 110.

Die Fläche weist an der dritten Schnittstelle 110 zumindest abschnittsweise eine raue Oberfläche auf. Die Fläche an der dritten Schnittstelle 110 weist einen Mittenrauwert auf, der größer als 1,5 µm ist, insbesondere der im Bereich von 3,5 µm bis 9 µm liegt. Dadurch hält ein Knochenzement sicher an der rauen Oberfläche der dritten Schnittstelle 110. Beispielsweise kennzeichnet ein demgegenüber kleinerer Mittenrauwert, z.B. ein Mittenrauwert Ra ≤ 1,5pm, eine glatte Oberfläche.

Es kann vorgesehen sein, dass die Fläche außerhalb der dritten Schnittstelle 110 eine glatte Oberfläche aufweist. Der Körper 104 weist an der dritten Schnittstelle 110 einen Hinterschnitt auf.

Die dritte Schnittstelle 110 umfasst einen Teil eines Umfangs des Körpers 104, der sich zwischen der ersten Schnittstelle 106 und der zweiten Schnittstelle 108 erstreckt. Der Hinterschnitt erstreckt sich im Beispiel entlang des Teils des Umfangs insbesondere über den gesamten Teil des Umfangs.

In Figur 1b ist eine Aufsicht auf das Augment 102 für das Knochenimplantat für die Tibia dargestellt. Die dritte Schnittstelle 110 weist eine erste Ausbuchtung 112 auf, die eine Form aufweist, die mit einer Form einer Seitenwand eines Befestigungselements des Knochenimplantats für die Tibia im Bereich der dritten Schnittstelle 110 korrespondiert. Die dritte Schnittstelle 110 weist eine zweite Ausbuchtung 114 in der ersten Ausbuchtung 112 auf, die eine Form aufweist, die mit einer Form einer Seitenwand eines Flügels des Knochenimplantats für die Tibia im Bereich der dritten Schnittstelle 110 korrespondiert.

In Figur 1c ist ein Schnitt A-A durch das Augment 102 für das Knochenimplantat für die Tibia an einer in Figur 1b mit A-A gekennzeichneten Schnittlinie dargestellt.

Die zweite Schnittstelle 108 erstreckt sich im Beispiel im Wesentlichen in einer Ebene. Der Hinterschnitt umfasst im Beispiel eine Wand des Körpers 104, die zumindest abschnittsweise in einem Winkel zwischen 89° und 45°, insbesondere zwischen 89° und 79°, zur Ebene angeordnet ist.

Der Hinterschnitt weist in einem Beispiel einen konischen oder konkaven Verlauf des Körper 104 von der zweiten Schnittstelle 108 zur ersten Schnittstelle 106 auf.

Der Körper 104 erstreckt sich zwischen der ersten Schnittstelle 106 und der zweiten Schnittstelle 108 in einer ersten Höhe 114. Der Hinterschnitt erstreckt sich im Beispiel zwischen der ersten Schnittstelle 106 und der zweiten Schnittstelle 108 in einer zweiten Höhe 116. Die zweite Höhe 116 ist kleiner als die erste Höhe 114. Die zweite Höhe 116 ist im Beispiel ein Bruchteil, insbesondere mindestens ein Sechstel oder mindestens ein Fünftel oder mindestens ein Viertel, der ersten Höhe 114.

Der Hinterschnitt erstreckt sich im Beispiel von der zweiten Höhe 116 bis zur ersten Schnittstelle 106.

Der Körper 104 verjüngt sich im Beispiel außerhalb der dritten Schnittstelle 110 von der ersten Schnittstelle 106 zur zweiten Schnittstelle 108. Der Körper 104 verjüngt sich im Beispiel von der ersten Schnittstelle 106 zur zweiten Schnittstelle 108. Es kann vorgesehen sein, dass sich der Körper 104 außerhalb der dritten Schnittstelle 110 von der ersten Schnittstelle 106 zur zweiten Schnittstelle 108 in einem Winkel von 90° zur Ebene erstreckt. Es kann vorgesehen sein, dass sich der Körper 104 nur außerhalb der dritten Schnittstelle 110 verjüngt.

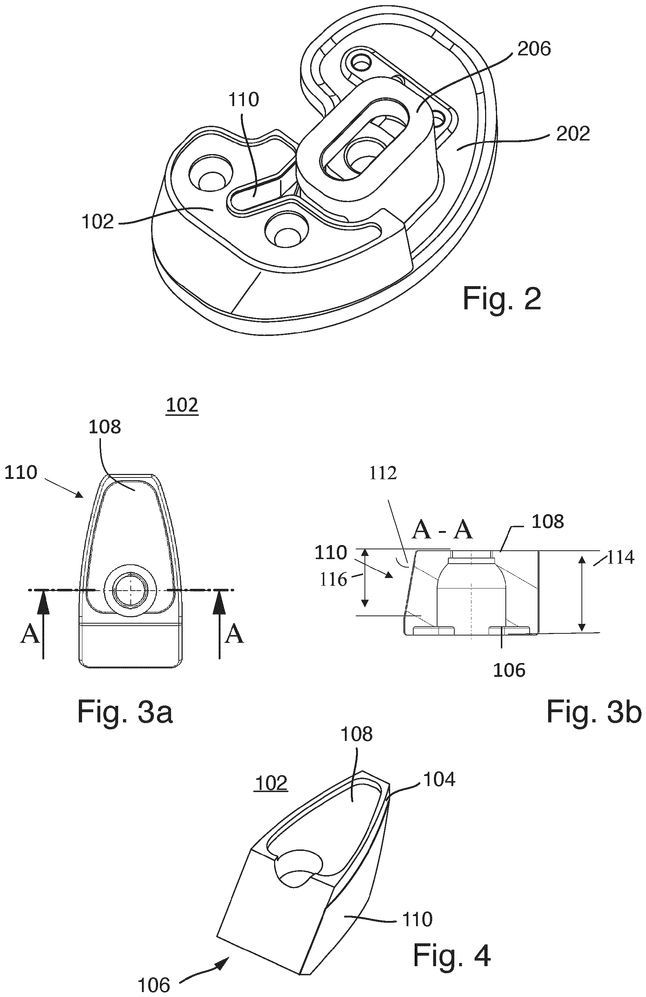

In Figur 2 ist eine schematische, perspektivische Ansicht des Augments 102 und wenigstens eines Teils des Knochenimplantats für die Tibia dargestellt.

Das Knochenimplantat umfasst ein Plateau 202 das auf einer Tibia, die mit einem horizontalen Sägeschnitt dafür präpariert ist, anordenbar ist. Das Knochenimplantat weist ein Befestigungselement 206 auf, das zur Verankerung des Knochenimplantats in einem dafür präparierten Markraum der Tibia ausgebildet ist.

Die dritte Schnittstelle 110 ist zur Aufnahme des Teils des Befestigungselements 206 ausgebildet. Das Befestigungselement 206 weist ein Kupplungselement mit einer Zentralachse auf. Das Kupplungselement ist im Beispiel eine Öffnung, in die ein zeichnerisch nicht näher dargestellter Schaft eingesetzt ist, der insbesondere eingeschraubt sein kann. In diesem Fall ist die Öffnung eine Gewindebohrung. Die Zentralachse dieser Gewindebohrung, d.h. des Kupplungselements, steht z.B. senkrecht oder im Wesentlichen senkrecht auf dem Plateau 202. Im Beispiel weisen die dritte Schnittstelle 110 und der Teil des Befestigungselements 206 miteinander korrespondierende Flächenformen und -größen auf. Zwischen der dritten Schnittstelle 110 und dem Befestigungselement 206 erstreckt sich z.B. eine spaltförmige Zementtasche, deren Spaltgrund das Plateau 202 bildet. In Richtung auf den Knochen, hier also die Tibia, ist die Zementtasche offen. Somit kann durch eine dort gebildete Einmündung Knochenzement in die Zementtasche eindringen und nach der Aushärtung in der Zementtasche verkeilen. Für dieses Verkeilen ist der Hinterschnitt an der dritten Schnittstelle 110 vorgesehen, deren Oberfläche sich in Richtung auf das Plateau 202 immer weiter von der verlängerten Achse der Gewindebohrung radial entfernt. Mithin kann vorgesehen sein, dass sich die Oberfläche der dritten Schnittstelle 110 über deren Höhe 116 in Richtung auf das Plateau 202 immer weiter von der verlängerten Achse des Kupplungselements entfernt.

In Figur 3a ist eine Aufsicht auf ein Augment 102 für ein Knochenimplantat für ein Femur dargestellt. Die dritte Schnittstelle 110 des Knochenimplantats für das Femur weist im Unterschied zur dritten Schnittstelle 110 des Knochenimplantats für die Tibia keine der für das Knochenimplantat für die Tibia beschriebenen Ausbuchtungen auf.

In Figur 3b ist ein Schnitt A-A durch das Augment 102 für das Knochenimplantat für das Femur an einer in Figur 3b mit A-A gekennzeichneten Schnittlinie dargestellt.

In Figur 4 ist eine schematische, perspektivische Ansicht des Augments 102 für das Knochenimplantat für das Femur dargestellt. Die dritte Schnittstelle 110 ist im Beispiel ausgebildet, neben einem Befestigungselement für ein Plateau des Knochenimplantats für das Femur angeordnet zu werden.

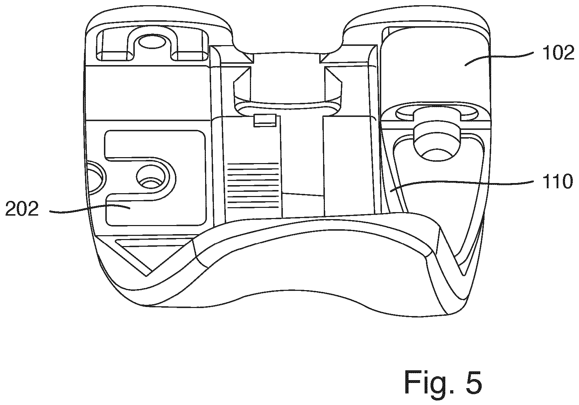

In Figur 5 ist eine schematische, perspektivische Ansicht des Augments 102 und wenigstens eines Teils des Knochenimplantats für das Femur dargestellt.

Das Plateau 202 ist auf einem Femur, das mit einem horizontalen Sägeschnitt dafür präpariert ist, anordenbar. Ein Teil des Plateaus 202 ist zur Verankerung des Knochenimplantats in einem dafür präparierten Markraum des Femurs ausgebildet.

Die dritte Schnittstelle 110 ist neben dem Teil des Plateau 202 angeordnet.

Die Tibia und das Femur sind Beispiele für Knochen, für die das Augment 102 anwendbar ist.

Das Augment 102 ist ausgebildet, einen Abstand zwischen dem Plateau 202 und dem Knochen zu füllen. Der Abstand ist beispielsweise ein Abstand zwischen einem ersten horizontalen Sägeschnitt zu einem demgegenüber distal zurückgesetzten zweiten horizontalen Sägeschnitt.

Das Plateau 202 und das Augment 102 sind im Beispiel miteinander an der ersten Schnittstelle 106 verbunden. Das Plateau 202 ist im Beispiel teilweise direkt mit dem Knochen verbunden. Das Augment 102 ist im Beispiel an der zweiten Schnittstelle 108 mit dem Knochen verbunden.

Das Plateau 202 und das Augment 102 sind im Beispiel ausgebildet, mittels Knochenzement mit dem Knochen verbunden zu werden. Die zweite Schnittstelle 108 und/oder die dritte Schnittelle 110 weisen im Beispiel eine raue Oberfläche zum Kontakt mit dem Knochenzement auf. Die zweite Schnittstelle 108 und/oder die dritte Schnittelle 110 weisen die raue Oberfläche vorzugsweise in Zementtaschen auf, die auf der Oberfläche des Augments 102 gebildet sind, insbesondere in der spaltförmigen Zementtasche.

Der Knochenzement wird im Beispiel im Hinterschnitt angeordnet. Durch Knochenzement im Hinterschnitt wird eine Fixierung des mit dem Augment 102 versehenen Knochenimplantats weiter erhöht. Die Augmente 102 sind bei einem Revisionschnitt gut zugänglich. Die Zementfixierung durch den Knochenzement im Hinterschnitt ist mit dem Revisionsschnitt einfach aufhebbar.

Für einen Operateur kann ein Satz von Augmenten 102 vorgesehen werden.

Der Satz von Augmenten 102 umfasst z.B. wenigstens zwei der beschriebenen Augmente 102, wobei sich der Körper 104 der wenigstens zwei Augmente 102 zwischen der ersten Schnittstelle 106 und der zweiten Schnittstelle 108 in unterschiedlichen Höhen erstreckt.

Der Satz von Augmenten 102 umfasst z.B. wenigstens zwei der beschriebenen Augmente 102, wobei die dritte Schnittstelle 110 zur Aufnahme unterschiedlich großer Befestigungselemente 206 ausgebildet ist.

Der Satz von Augmenten 102 umfasst z.B. wenigstens zwei der beschriebenen Augmente 102, wobei die Hinterschnitte sich in ihrer Form oder im Winkel unterscheiden.

In einem Beispiel umfasst der Satz ein erstes Augment 102 und ein zweites Augment 102 auf, die wie für das Knochenimplantat beschrieben ausgeführt sind. Das erste Augment 102 und das zweite Augment 102 sind aneinander anordenbar ausgeführt. Die erste Schnittstelle 106 zur Befestigung des ersten Augments 102 am Plateau 202 des Knochenimplantats und die zweite Schnittstelle 108 zur Befestigung des zweiten Augments 102 am Knochen sind ausgebildet, das erste Augment 102 und das zweite Augment 102 zu verbinden. Dadurch sind die Augmente 102 in einem Stapel stapelbar. Dadurch können unterschiedliche Differenzen ausgefüllt werden.

Es kann vorgesehen sein, dass das zweite Augment 102 ohne Hinterschnitt an seiner dritten Schnittstelle 110 zum Befestigungselement 206 ausgebildet ist. Die Zementfixierung ist z.B. in einem dem Knochen am nächsten angeordneten Augment 102 des Stapels vorgesehen.

## Patentansprüche

1. Augment (102) für ein Knochenimplantat insbesondere für eine Tibia oder ein Femur, **dadurch gekennzeichnet, dass** das Augment (102) einen Körper (104) aufweist, wobei der Körper (104) eine Schnittstelle (106) zur Befestigung des Augments (102) an einem Plateau (202) des Knochenimplantats, eine Schnittstelle (108) zur Befestigung des Augments (102) am Knochen und eine Schnittstelle (110 )zu einem Befestigungselement (206) zur Verankerung des Knochenimplantats in einem Markraum eines Knochens, insbesondere der Tibia oder des Femurs, aufweist, die sich zwischen der Schnittstelle (106) zur Befestigung des Augments (102) am Plateau (202) und der Schnittstelle (108) zur Befestigung des Augments (102) am Knochen erstreckt, wobei der Körper (104) an der Schnittstelle (110) zum Befestigungselement (206) einen Hinterschnitt aufweist.

2. Augment (102) nach Anspruch 1, **dadurch gekennzeichnet, dass** der Hinterschnitt einen konischen oder konkaven Verlauf des Körpers (104) von der Schnittstelle (108) zur Befestigung des Augments (102) am Knochen zur Schnittstelle (106) zur Befestigung des Augments (102) am Plateau (202) umfasst.

3. Augment (102) nach einem der vorherigen Ansprüche, **dadurch gekennzeichnet, dass** sich der Körper (104) zwischen der Schnittstelle (106) zur Befestigung des Augments (102) am Plateau (202) und der Schnittstelle (108) zur Befestigung des Augments (102) am Knochen in einer ersten Höhe erstreckt, wobei sich der Hinterschnitt zwischen der Schnittstelle (106) zur Befestigung des Augments (102) am Plateau (202) und der Schnittstelle (108) zur Befestigung des Augments (102) am Knochen in einer zweiten Höhe (116) erstreckt, die kleiner als die erste Höhe (114) ist, insbesondere von der zweiten Höhe (116) bis zur Schnittstelle (106) zur Befestigung des Augments (102) am Plateau (202).

4. Augment (102) nach einem der vorherigen Ansprüche, **dadurch gekennzeichnet, dass** die Schnittstelle (108) zur Befestigung des Augments (102) am Knochen sich im Wesentlichen in einer Ebene erstreckt, wobei der Hinterschnitt eine Wand des Körpers (104) umfasst, die zumindest abschnittsweise in einem Winkel zwischen 89° und 45°, insbesondere zwischen 89° und 79°, zur Ebene angeordnet ist.

5. Augment (102) nach einem der vorherigen Ansprüche, **dadurch gekennzeichnet, dass** der Körper (104) sich außerhalb der Schnittstelle (110) zum Befestigungselement (206) von der Schnittstelle (106) zur Befestigung des Augments (102) am Plateau (202) zur Schnittstelle (108) zur Befestigung des Augments (102) am Knochen verjüngt oder dass die Schnittstelle (108) zur Befestigung des Augments (102) am Knochen sich im Wesentlichen in der Ebene erstreckt, wobei der Körper (104) sich außerhalb der Schnittstelle (110) zum Befestigungselement (206) von der Schnittstelle (106) zur Befestigung des Augments (102) am Plateau (202) zur Schnittstelle (108) zur Befestigung des Augments (102) am Knochen im Winkel von 90° oder im Wesentlichen von 90° zur Ebene erstreckt.

6. Augment (102) nach einem der vorherigen Ansprüche, **dadurch gekennzeichnet, dass** der Körper (104) sich von der Schnittstelle zur Befestigung des Augments (102) am Plateau (202) zur Schnittstelle zur Befestigung des Augments (102) am Knochen verjüngt.

7. Augment (102) nach einem der vorherigen Ansprüche, **dadurch gekennzeichnet, dass** der Körper (104) eine zwischen der Schnittstelle (106) zur Befestigung des Augments (102) am Plateau (202) und der Schnittstelle (108) zur Befestigung des Augments (102) am Knochen umlaufende Fläche umfasst, wobei die Fläche die Schnittstelle (110) zum Befestigungselement (206) umfasst.

8. Augment (102) nach Anspruch 7, **dadurch gekennzeichnet, dass** die Fläche an der Schnittstelle (110) zum Befestigungselement (206) zumindest abschnittsweise eine raue Oberfläche aufweist.

9. Augment (102) nach Anspruch 7 oder 8, **dadurch gekennzeichnet, dass** die Fläche außerhalb der Schnittstelle (110) zum Befestigungselement (206) eine glatte Oberfläche aufweist.

10. Augment (102) nach einem der vorherigen Ansprüche, **dadurch gekennzeichnet, dass** die Schnittstelle (110) zum Befestigungselement (206) einen Teils eines Umfangs des Körpers (104) umfasst, der sich zwischen der Schnittstelle (106) zur Befestigung des Augments (102) am Plateau (202) und der Schnittstelle (108) zur Befestigung des Augments (102) am Knochen erstreckt, wobei sich der Hinterschnitt entlang des Teils des Umfangs insbesondere über den gesamten Teil des Umfangs erstreckt.

11. Satz von Augmenten (102), **dadurch gekennzeichnet, dass** der Satz von Augmenten (102) wenigstens zwei Augmente (102) nach einem der Ansprüche 1 bis 10 umfasst, wobei sich der Körper (104) der wenigstens zwei Augmente (102) zwischen der Schnittstelle (106) zur Befestigung des Augments (102) am Plateau (202) und der Schnittstelle (108) zur Befestigung des Augments (102) am Knochen in unterschiedlichen Höhen erstreckt oder die Schnittstelle (110) zum Befestigungselement (206) für unterschiedlich große Befestigungselemente (206) ausgebildet ist, oder sich die Hinterschnitte in ihrer Form oder im Winkel unterscheiden.

12. Satz von Augmenten (102) nach Anspruch 11, **dadurch gekennzeichnet, dass** der Satz ein erstes Augment (102) und ein zweites Augment (102) nach einem der Ansprüche 1 bis 10 umfasst, wobei das erste Augment (102) und das zweite Augment (102) aneinander anordenbar sind, wobei die Schnittstelle (106) zur Befestigung des ersten Augments (102) am Plateau (202) des Knochenimplantats und die Schnittstelle (108) zur Befestigung des zweiten Augments (102) am Knochen ausgebildet sind, das erste Augment (102) und das zweite Augment (102) zu verbinden.

13. Satz von Augmenten (102) nach Anspruch 12, **dadurch gekennzeichnet, dass** das zweite Augment (102) ohne Hinterschnitt an seiner Schnittstelle (110) zum Befestigungselement (206) ausgebildet ist.
